# EUROPÄISCHE PATENTSCHRIFT

(11) **EP 1 136 038 B1**
(45) Veröffentlichungstag und Bekanntmachung des Hinweises auf die Patenterteilung: **28.07.2004**
(21) Anmeldenummer: 00119191.5
(22) Anmeldetag: 05.09.2000
(51) Int. Cl.: A61B 5/12

(54) **Gerät zum elektromechanischen Stimulieren und Prüfen des Gehörs**
Apparatus for the electromechanical stimulation and testing of hearing
Appareil pour la stimulation électromécanique et tester l'ouie

(30) Priorität: 22.03.2000 DE 10014200
(43) Veröffentlichungstag der Anmeldung: 26.09.2001
(73) Patentinhaber: PHONAK AG, 8712 Stäfa (CH)
(72) Erfinder: Reischl, Gabriele, 81541 München (DE); Leysieffer, Hans, 82024 Taufkirchen (DE); Müller, Gerd M., Dr.rer.nat.Dipl.-Phys., 85716 Lohhof (DE)
(74) Vertreter: Schwan, Gerhard, Dipl.-Ing.

(56) Entgegenhaltungen:
- DE-A- 3 121 429
- DE-C- 19 821 602
- US-A- 5 776 144
- US-A- 5 833 626

## Beschreibung

Die Erfindung bezieht sich auf ein Gerät zum elektromechanischen Stimulieren und Prüfen des Gehörs, mit einem elektromechanischen Wandler zum Erzeugen von mechanischen Schwingungen, einem mechanischen Koppelelement zum Übertragen von mechanischen Stimulationsschwingungen von dem elektromechanischen Wandler ohne operativen Eingriff durch den äußeren Gehörgang auf den Umbo und damit auf den Hammergriff der Ossikelkette, einer Positioniereinrichtung zum Positionieren des Koppelelements mit Bezug auf den Umbo, sowie einer Fixiereinrichtung für die feste, spielfreie Anbindung der Positioniereinrichtung an den menschlichen Körper, insbesondere den menschlichen Schädel.

Ein Gerät dieser Art ist aus US-A-5 833 626 bekannt. Eine dafür geeignete Positioniereinrichtung ist in US-A-5 776 144 näher erläutert. Ein solches Gerät erlaubt es, auf nichtinvasive Weise, das heißt ohne operativen Eingriff, dem zur Implantation anstehenden Probanden die Hörverbesserung und Klangqualität präoperativ zu demonstrieren, die durch den Einsatz eines teil- oder vollimplantierbaren Hörsystems zu erwarten ist, welches durch Ankopplung eines elektromechanischen Wandlers das Mittel- oder Innenohr mit mechanischen Schwingungen stimuliert. Ein vollimplantierbares Hörsystem dieser Art ist unter anderem in dem Aufsatz von H. Leysieffer et al. "Ein vollständig implantierbares Hörsystem für Innenohrschwerhörige: TICA LZ 3001" HNO, 13d. 46, Oktober 1998, Seiten 853 bis 863 beschrieben.

Die Positioniereinrichtung erlaubt es, das Wirkende des mechanischen Koppelelements, beispielsweise einer mit dem elektromechanischen Wandler verbundenen Koppelstange, präzise an den Zielort heranzuführen. Mittels der Fixiereinrichtung wird die räumliche Lage zwischen dem positionierten Koppelelement und dem Umbo fixiert. Praktische Erfahrungen haben gezeigt, daß ein gewisses Risiko beim Einsatz des Gerätes darin besteht, daß die Positionier- und/oder die Fixiereinrichtung versehentlich berührt und bewegt werden. Dadurch kann eine Verletzung des Trommelfells oder der Ossikelkette erfolgen, oder auf das Innenohr und die Bogengänge kann ein Druck ausgeübt werden.

Dies kann zu Schwindel oder gar Hörverlust führen. Ein solches Risiko besteht auch bei spontanen Kopfbewegungen des Probanden.

Der Erfindung liegt die Aufgabe zugrunde, die Sicherheit eines Gerätes der eingangs genannten Art weiter zu erhöhen.

Diese Aufgabe wird erfindungsgemäß dadurch gelöst, daß bei einem solchen Gerät zwischen der Positioniereinrichtung und dem elektromechanischen Wandler ein Zwischenglied angeordnet ist, das so ausgelegt und bemessen ist, daß es quasistationäre Positioniereinstellungen von der Positioniereinrichtung zu dem elektromechanischen Wandler überträgt, aber eine Übertragung mindestens von dynamischen Kräften von der Positioniereinrichtung auf das Koppelelement mindestens so weit herabsetzt, daß das Risiko einer Mittel- oder Innenohrschädigung deutlich gemindert ist.

Bei dem Stimulations- und Prüfgerät nach der Erfindung folgt der Wandler samt Koppelelement den relativ langsamen, vorliegend als quasistationär bezeichneten Lageänderungen, die durch Betätigen der Positioniereinrichtung bewirkt werden. Der Arzt kann damit das Wirkende des Koppelelements präzise und frei von Relativbewegungen zu Strukturen im menschlichen Körper, insbesondere an den Umbo, als Zielpunkt heranführen. Dagegen werden bei einer unbeabsichtigten, in der Regel ruck- oder stoßweise erfolgenden äußeren Einwirkung, beispielsweise durch Anstoßen an der Positioniereinrichtung mit der Hand, einem Instrument oder dergleichen, auf die Positioniereinrichtung einwirkende dynamische Kräfte von dem Wandler und dem Koppelelement mindestens in erheblichem Umfang ferngehalten.

Bevorzugte Ausführungsformen der Erfindung ergeben sich aus den Unteransprüchen.

Eine konstruktiv besonders einfache Lösung wird erhalten, wenn das Zwischenglied als Federglied ausgeführt ist. Dabei hat das das Federglied sowie den elektromechanischen Wandler und das Koppelelement einschließende Feder/Massesystem vorzugsweise eine Eigenfrequenz im Bereich von 0,5 bis 5 Hz.

Das Federglied kann aus einer oder mehreren Biegefedern, insbesondere einfachen Biegefedern, bestehen.

Vorteilhaft ist das Koppelelement als Koppelstange ausgebildet und das Federglied mindestens näherungsweise senkrecht zu der Längsachse der Koppelstange ausgerichtet.

Die Erfindung wird nachfolgend unter Bezugnahme auf die beiliegende Zeichnung näher beschrieben. Die einzige Figur zeigt eine schematische Ansicht des Stimulations- und Prüfgerätes nach der Erfindung.

Das Gerät weist einen elektromechanischen Wandler 1 auf, mit dessen Ausgangsseite ein Koppelelement in Form einer starren Koppelstange 2 fest verbunden ist. Der Wandler 1 steht über ein Zwischenglied 3 mit einer insgesamt mit 4 bezeichneten Positioniereinrichtung in Verbindung. Die Positioniereinrichtung 4 ist ihrerseits an einer bei 5 nur angedeuteten Fixiereinrichtung angebracht, die es erlaubt, die Positioniereinrichtung 4 am menschlichen Körper, insbesondere am menschlichen Schädel, fest und spielfrei anzubinden.

Die veranschaulichte Positioniereinrichtung 4 ist mit einer an die Fixiereinrichtung 5 angekoppelten Basis 7 versehen. Die Basis 7 trägt ein klemmbares Kugelgelenk 8, das eine Gelenkkugel 9 und eine zugehörige Kugelaufnahme 10 aufweist. Mittels einer Klemmschraube 11 läßt sich das Kugelgelenk 8 in einer Lage blockieren, die mittels einer mit der Gelenkkugel 9 fest verbundenen Stellstange 12 eingestellt werden kann. An der Stellstange 12 ist ein in seiner Länge einstellbarer, querverlaufender Tragarm 14 angebracht. Die eingestellte Länge des Tragarms 14 wird mittels einer Klemmschraube 15 fixiert. An dem von der Stellstange abliegenden Ende des Tragarms 14 greift eine Linear-Stellvorrichtung 16 an. Diese ist an ihrem in der Figur unteren Ende mit einem Schieber 17 verbunden, an dem ein Führungsbolzen 18 angebracht ist. Der Führungsbolzen 18 ist in einer Bohrung 19 des Tragarms 14 in einer zur Längsachse der Koppelstange 2 im wesentlichen parallelen Richtung verschiebbar geführt. Der Wandler 1 steht mit dem Schieber 17 über das Zwischenglied 3 in Verbindung. Mittels der Linear-Stellvorrichtung 16 kann der Wandler 1 über den Schieber 17 und das Zwischenglied 3 in Längsrichtung der Koppelstange 2 feinfühlig verstellt werden. Die Linear-Stellvorrichtung 16 kann insbesondere eine nicht näher dargestellte hydraulische Kolben/Zylinder-Anordnung aufweisen, die bei Betätigung an ihrem von dem Wandler 1 abliegenden Ende eine Feineinstellung des Wandlers 1 samt Koppelstange 2 gegenüber dem Tragarm 14 in der zu diesem im wesentlichen senkrechten Richtung erlaubt.

An der Basis 7 ist ferner ein Ohrtrichter 20 leicht abnehmbar angebracht. Zum Festklemmen und Lösen des Ohrtrichters 20 dient eine mit der Basis 7 und dem Ohrtrichter 20 zusammenwirkende Klemme 22. Der Ohrtrichter 20 nimmt den von dem Wandler 1 abliegenden Teil der Koppelstange 2 auf, wobei die Längsachse der Koppelstange 2 mit der Längsachse des Ohrtrichters ausgerichtet werden kann. Gegebenenfalls kann der Ohrtrichter 20 zum Ausgleichen geringer räumlicher Winkel kardanisch an der Basis 7 gelagert sein.

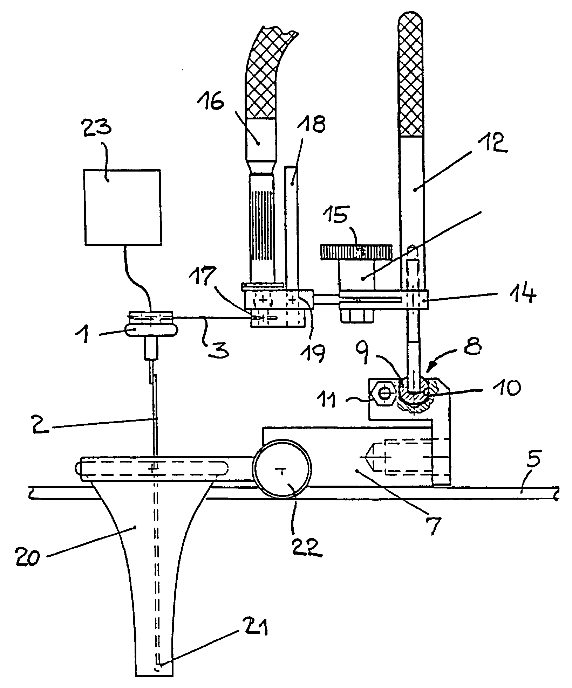

Bei Verwendung des Gerätes wird der Ohrtrichter 18 unter visueller Kontrolle, bei Bedarf unter Zuhilfenahme eines Mikroskops, in den äußeren Gehörgang des Probanden eingeführt. In der entsprechenden Stellung wird die Basis 7 mittels der Fixiereinrichtung 5 mit Bezug auf den Schädel des Probanden festgelegt. Die Fixiereinrichtung 5 kann beispielsweise als Kopfhalter zur beidseitigen schmerzfreien Befestigung im Ohrbereich in der aus Fig. 4 der US-A-5 776 144 bekannten Art ausgebildet sein und auf der dem Ohrtrichter 20 gegenüberliegenden Schädelseite ein Formteil aufweisen, das die dort befindliche Ohrmuschel als zweite Auflagefläche umschließt. Als Fixiereinrichtung 5 kann unter anderem aber auch ein entsprechend angepaßter Helm vorgesehen sein, an dem die Basis 7 der Positioniereinrichtung 4 befestigt ist.

Bei gelöster Klemmung des Kugelgelenks 8 läßt sich die Linear-Stellvorrichtung 16 um das Zentrum der Gelenkkugel 9 in allen drei rotatorischen Freiheitsgraden des Raums drehen. Der gegenseitige Abstand der Längsachsen von Stellstange 12 und Koppelstange 2 kann bei gelöster Klemmschraube 15 eingestellt werden. Durch Befestigen der Fixiereinrichtung 5 am Körper des Probanden, Positionieren des an der Fixiereinrichtung befestigten Systems, nachfolgende Klemmung der Klemmschrauben 11 und 15 und entsprechendes Einstellen der Linear-Stellvorrichtung 16 ist somit eine exakte, spielfreie Positionierung des zweckmäßig kugeligen freien Wirkendes 21 der Koppelstange 2 zum Umbo als körperfestem Zielpunkt möglich. Die Position des freien Wirkendes 21 kann dabei zum Beispiel durch ein Mikroskop kontrolliert werden. Durch das gegenseitige Versetzen von Koppelstange 2 und Positioniereinrichtung 4 ist sichergestellt, daß die optische Achse des Mikroskopes bzw. des bloßen Auges des Arztes nicht vom Positioniersystem selbst oder dessen Bestandteilen verdeckt wird. Die Stimulation erfolgt dadurch, daß ein bei 23 schematisch angedeuteter Signalgenerator elektrische Signale an den elektromechanischen Wandler 1 liefert und der Wandler 1 über die Koppelstange 2 den Umbo zu mechanischen Schwingungen anregt. Der Wandler 1 kann dabei vorzugsweise dem elektromechanischen Wandler des zu implantierenden Hörsystems entsprechen. Ein Beispiel für einen geeigneten elektromechanischen Wandler ist im einzelnen in den Aufsätzen von H. Leysiffer et al. "Ein implantierbarer Hörgerätewandler für Innenohrschwerhörige" Teil I und Teil II, HNO, Bd. 45, Oktober 1997, Seiten 792 bis 815, beschrieben.

Bei dem veranschaulichten Ausführungsbeispiel besteht das Zwischenglied 3 aus zwei parallel angeordneten einfachen Biegefedern, von denen in der Figur nur die eine zu erkennen ist, während die andere senkrecht zur Bildebene versetzt dahinter verläuft. Das Feder/Massesystem, welches das Zwischenglied 3 sowie den elektromechanischen Wandler 1 und die Koppelstange 2 einschließt, ist vorzugsweise so ausgelegt, daß es eine Eigen- oder Resonanzfrequenz (beziehungsweise im Falle mehrerer Eigenfrequenzen eine niedrigste erste Eigenfrequenz) im Bereich von 0,5 bis 5 Hz hat. Dadurch werden dynamische Kräfte mit höherer als dieser Eigenfrequenz, die zum Beispiel durch versehentliche Stöße auf die Positioniereinrichtung 4 auftreten können, wenn überhaupt, nur wesentlich abgeschwächt von der Positioniereinrichtung 4 auf die Koppelstange 2 übertragen. Die Koppelstange 2 macht jedoch normalerweise die quasistationären Positioniereinstellungen der Positioniereinrichtung 4 mit. Wenn allerdings beim Positionieren der Wandler 1 dem Zielort versehentlich zu weit angenähert wird, können die das Zwischenglied 3 bildenden Biegefedern ausweichen und auch dadurch einer Beschädigung von Mittel- und/oder Innenohr entgegenwirken.

Es versteht sich, daß das Zwischenglied 3 grundsätzlich auch in anderer Weise aufgebaut sein kann. Zum Beispiel kann das Zwischenglied 3 einen Kraftbegrenzer, beispielsweise in Form einer Reibungs- oder Induktionskupplung, enthalten, der Kräfte nur bis zu einem vorgegebenen oberen Grenzwert durchläßt.

## Patentansprüche

1. Gerät zum elektromechanischen Stimulieren und Prüfen des Gehörs, mit einem elektromechanischen Wandler (1) zum Erzeugen von mechanischen Schwingungen, einem mechanischen Koppelelement (2) zum Übertragen von mechanischen Stimulationsschwingungen von dem elektromechanischen Wandler ohne operativen Eingriff durch den äußeren Gehörgang auf den Umbo und damit auf den Hammergriff der Ossikelkette, einer Positioniereinrichtung (4) zum Positionieren des Koppelelements mit Bezug auf den Umbo, sowie einer Fixiereinrichtung (5) für die feste, spielfreie Anbindung der Positioniereinrichtung an den menschlichen Körper, insbesondere den menschlichen **Schädel, dadurch gekennzeichnet, daß** zwischen der Positioniereinrichtung (4) und dem elektromechanischen Wandler (1) ein Zwischenglied (3) angeordnet ist, das so ausgelegt und bemessen ist, daß es quasistationäre Positioniereinstellungen von der Positioniereinrichtung zu dem elektromechanischen Wandler überträgt, aber eine Übertragung mindestens von dynamischen Kräften von der Positioniereinrichtung auf das Koppelelement mindestens so weit herabsetzt, daß das Risiko einer Mittel- oder Innenohrschädigung deutlich gemindert ist.

2. Gerät nach Anspruch 1, **dadurch gekennzeichnet, daß** das Zwischenglied (3) als Federglied ausgeführt ist.

3. Gerät nach Anspruch 2, **dadurch gekennzeichnet, daß** das das Federglied (3) sowie den elektromechanischen Wandler (1) und das Koppelelement (2) einschließende Feder/Massesystem eine Eigenfrequenz im Bereich von 0,5 bis 5 Hz hat.

4. Gerät nach Anspruch 2 oder 3, **dadurch gekennzeichnet, daß** das Federglied (3) aus einer oder mehreren Biegefedern besteht.

5. Gerät nach Anspruch 4, **dadurch gekennzeichnet, daß** das Federglied (3) aus einer oder mehreren einfachen Biegefedern besteht.

6. Gerät nach einem der Ansprüche 2 bis 5, **dadurch gekennzeichnet, daß** das Koppelelement (2) als Koppelstange ausgebildet ist und das Federglied (3) mindestens näherungsweise senkrecht zu der Längsachse der Koppelstange ausgerichtet ist.

## Claims

1. Device for electromechanical stimulation and testing of hearing, comprising an electromechanical transducer (1) for producing mechanical vibrations, a mechanical coupling element (2) for transmitting without surgery mechanical stimulation vibrations from the electromechanical transducer through the external auditory canal to the umbo and hence to the manubrium mallei of the ossicular chain, positioning (4) means for positioning the coupling element with reference to the umbo, and fixing means (5) for secure, play-free linkage of the positioning means to the human body, in particular to the human skull, **characterised in that** an intermediate element (3) is provided between the positioning means (4) and the electromechanical transducer (1), said intermediate element being configured and dimensioned for transmitting quasi-steady-state positioning adjustments from the positioning means to the electromechanical transducer, and for reducing transmission of at least dynamic forces from the positioning means to the coupling element at least to such an extent that the risk of middle or inner ear damage is substantially reduced.

2. Device as claimed in claim 1, **characterised in that** the intermediate element (3) is a spring member.

3. Device as claimed in claim 2, **characterised in that** the spring/mass system including said spring member (3), said electromechanical transducer (1), and said coupling element (2) has a natural frequency in the range of from 0.5 to 5 Hz.

4. Device as claimed in claim 2 or 3, **characterised in that** the spring member (3) comprises one or more flexional springs.

5. Device as claimed in claim 4, **characterised in that** the spring member (3) comprises one or more simple flexional springs.

6. Device as claimed in any one of claims 2 to 5, **characterised in that** the coupling element (2) is a coupling rod and the spring member (3) is aligned at least roughly perpendicular to the longitudinal axis of the coupling rod.

## Revendications

1. Appareil pour la stimulation électromécanique et la vérification de l'ouïe, avec un convertisseur électromécanique (1) pour produire des vibrations mécaniques, un élément de couplage (2) mécanique pour la transmission à travers le conduit auditif externe sur l'ombilic, et ainsi sur le manche du marteau de la chaîne ossiculaire, des vibrations de stimulation mécaniques provenant du convertisseur électromécanique sans intervention opératoire, un dispositif de positionnement (4) pour le positionnement de l'élément de couplage par rapport à l'ombilic, ainsi qu'un dispositif de fixation (5) pour le raccordement solide et sans jeu du dispositif de positionnement au corps humain, en particulier au crâne humain, **caractérisé en ce qu'**un membre intermédiaire (3) est disposé entre le dispositif de positionnement (4) et le convertisseur électromécanique (1), ledit membre intermédiaire étant d'une réalisation et de dimensions telles qu'il transmet des réglages de positionnement quasi stationnaires du dispositif de positionnement au convertisseur électromécanique, mais réduit tellement une transmission d'au moins de forces dynamiques provenant du dispositif de positionnement sur l'élément de couplage que le risque d'une lésion de l'oreille moyenne ou de l'oreille interne est considérablement réduit.

2. Appareil selon la revendication 1, **caractérisé en ce que** le membre intermédiaire (3) est réalisé en tant que membre à ressorts.

3. Appareil selon la revendication 2, **caractérisé en ce que** le système ressort/masse comprenant le membre à ressorts (3) ainsi que le convertisseur électromécanique (1) et l'élément de couplage (2) a une fréquence propre comprise dans une plage allant de 0,5 à 5 Hz.

4. Appareil selon la revendication 2 ou 3, **caractérisé en ce que** le membre à ressorts (3) est composé d'un ou de plusieurs ressorts spiraux.

5. Appareil selon la revendication 4, **caractérisé en ce que** le membre à ressorts (3) est composé d'un ou de plusieurs ressorts spiraux simples.

6. Appareil selon l'une quelconque des revendications 2 à 5, **caractérisé en ce que** l'élément de couplage (2) est réalisé en tant que tige de couplage et le membre à ressorts (3) est orienté au moins approximativement perpendiculairement à l'axe longitudinal de la tige de couplage.
